# FASCICULE DE BREVET EUROPEEN

(11) **EP 1 255 827 B1**
(45) Date de publication et mention de la délivrance du brevet: **04.10.2006**
(21) Numéro de dépôt: 01907764.3
(22) Date de dépôt: 09.02.2001
(51) Int. Cl.: C12N 15/11, A61K 31/12, A61K 31/7125

(54) **OLIGONUCLEOTIDES ET LEUR UTILISATION POUR MODULER L'EXPRESSION DE LA TYROSINASE INTERVENANT DANS LA SYNTHESE DE PIGMENTS MELANIQUES**
OLIGONUKLEOTIDE UND DEREN VERWENDUNG UM DIE EXPRESSION DER TYROSINASE, DIE IN DER SYNTHESE VON MELANISCHEN PIGMENTEN BETEILIGT IST, ZU MODULIEREN
OLIGONUCLEOTIDES AND THEIR USE FOR MODULATING THE EXPRESSION OF TYROSINASE INVOLVED IN THE SYNTHESIS OF MELANIC PIGMENTS

(30) Priorité: 11.02.2000 FR 0001730
(43) Date de publication de la demande: 13.11.2002
(73) Titulaire: LVMH RECHERCHE, 75008 Paris (FR)
(72) Inventeur: KURFURST, Robin, F-45160 Olivet (FR); JOLY, Régine, 80600 AUTHEUX (FR)
(74) Mandataire: Ahner, Francis
(86) Numéro de dépôt international: PCT/FR2001/000398
(87) Numéro de publication internationale: WO 2001/058918

(56) Documents cités:
- EP-A- 0 714 907
- EP-A- 0 895 779
- WO-A-95/07924
- WO-A-95/34280
- WO-A-97/14709
- WO-A-99/25819
- US-A- 5 078 989
- ZHAO, H. ET AL.: "Retroviral infection with human tyrosinase-related protein-1 (TRP-1) cDNA upregulates tyrosinase activity and melanin synthesis in a TRP-1-deficient melanoma cell line." JOURNAL OF INVESTIGATIVE DERMATOLOGY, vol. 106, no. 4, 1996, pages 744-752, XP000953024 ISSN: 0022-202X
- BOISSY, R. ET AL.: "Human tyrosinase related protein-1 (TRP-1) does not function as a DHICA oxidase activity in contrast to murine TRP-1." EXP DERMATOL 1998 AUG;7(4):198-204, XP000953013
- UHLMANN E ET AL: "ANTISENSE OLIGONUCLEOTIDES: A NEW THERAPEUTIC PRINCIPLE" CHEMICAL REVIEWS, vol. 90, no. 4, 1 juin 1990 (1990-06-01), pages 543-584, XP000141412 ISSN: 0009-2665 cité dans la demande

## Description

La présente invention concerne des nouvelles séquences oligonucléotides ainsi que leurs dérivés.

Ces nouvelles séquences oligonucléotides sont capables de s'hybrider avec le gène ou avec un produit du gène codant pour l'une des enzymes essentielles intervenant dans la voie de synthèse de pigments mélaniques.

La présente invention concerne également l'utilisation de ces nouvelles séquences oligonucléotides comme agents dépigmentants ou blanchissant de la peau dans une composition cosmétique ou dans une composition dermatologique.

Chez l'homme, la pigmentation résulte de la synthèse et de la distribution des pigments mélaniques dans la peau, les follicules pileux ou les yeux. La pigmentation est génétiquement prédéfinie mais elle est régulée par de nombreux facteurs internes ou externes. Les mélanines produites par les mélanocytes ainsi que le nombre de mélanocytes, leur activité tyrosinasique et leur capacité à exporter les mélanines vers les kératinocytes, la taille des mélanosomes qui contiennent des grains de mélanine, vont conditionner la couleur de la peau humaine. Pour chaque individu, la couleur de la peau varie principalement en fonction de l'irradiation plus ou moins importante des rayons ultra-violets (UV). Autrement dit pour chaque individu, il existe une pigmentation cutanée de base lorsqu'il subit la plus faible irradiation UV, correspondant à sa couleur de peau la plus claire, et une pigmentation cutanée plus intense s'il reçoit une irradiation UV plus forte, allant jusqu'à une pigmentation maximum correspondant à sa couleur de peau la plus foncée lorsqu'il est exposé de manière prolongée à une irradiation UV intense, telle que celle que l'on peut rencontrer en altitude en montagne.

D'autre part, comme on le sait bien, il existe dans la population mondiale, une très grande diversité génétique quant à la pigmentation cutanée. Ainsi, selon les populations, la couleur de la peau correspondant à la pigmentation de base définie ci-dessus, présente une teinte plus ou moins claire se situant entre les deux extrêmes : très claire et très foncée. Egalement selon les populations, la différence de teinte de la peau entre la pigmentation de base et la pigmentation maximum est plus ou moins importante. Ainsi, il est bien connu que les personnes appartenant à certaines populations à peau claire (pigmentation de base) réagissent rapidement et/ou de manière importante à l'action des UV et peuvent donc facilement présenter une peau de teinte foncée, même lorsque ces personnes ne se sont pas exposées volontairement et de façon prolongée au soleil. Dans la suite du texte, ces personnes seront désignées par l'expression « personnes très réactives aux UV ». Il en est notamment ainsi de populations d'origine asiatique ou de certaines populations dites métisses.

Par ailleurs, des personnes voient apparaître sur leur peau, en particulier au niveau du visage ou des mains des zones ou des taches plus foncées ou plus colorées conférant à la peau une hétérogénéité. Ces taches sont dues à une concentration importante de mélanine dans les kératinocytes de l'épiderme.

Le mécanisme de formation de la pigmentation cutanée fait intervenir la synthèse des mélanines. Ce mécanisme est particulièrement complexe et fait intervenir schématiquement les principales étapes suivantes :
Tyrosine 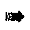 Dopa 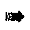 Dopaquinone 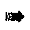 Dopachrome 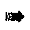 Mélanines

Les enzymes connues intervenant dans cette suite de réactions sont essentiellement la tyrosinase et la tyrosinase-related-protein 1 (TRP-1). Ces enzymes catalysent notamment la réaction de transformation de la tyrosine en Dopa (Dihydroxyphénylalanine) et la réaction de transformation de la Dopa en Dopaquinone conduisant à la formation des pigments mélaniques. Ces enzymes n'interviennent pas individuellement au cours du mécanisme réactionnel de la mélanogenèse. En effet, la tyrosinase et la TRP-1 forment un complexe enzymatique (Winder *et al.* (1994) Cell. Mol. Biol. Res. 40 : 7-8 ; Orlow *et al.* (1994) J. of Investigative Dermatology 103: 196-211). Ces deux enzymes agissent donc de concert et il semble que ces deux enzymes ne fonctionnent jamais l'une sans l'autre. Il est connu que, lorsqu'une cellule est déplétée en TRP-1, une perte de l'activité tyrosinase est observée (Orlow *et al.* (1994)) ; ceci signifie que la tyrosinase pour être active requiert la présence de TRP-1 (Zhao et *al.* (1996) J. of Investigative Dermatology 106 : 744-752).

Ces enzymes essentielles intervenant dans le voie de synthèse des mélanines seront désignées dans la suite du présent texte par l'expression "enzymes essentielles de la pigmentation".

Une molécule est reconnue comme dépigmentante si elle agit directement sur les mélanocytes épidermiques en inhibant l'activité de ces cellules et/ou si elle bloque l'une des étapes de la biosynthèse des mélanines. C'est le cas notamment lorsque la molécule inhibe l'une des enzymes impliquées dans la mélanogénèse ou lorsqu'elle réagit avec les composés chimiques de la chaîne de synthèse des mélanines.

Les substances dépigmentantes connues sont notamment l'hydroquinone et ses dérivés, l'acide ascorbique et ses dérivés, des extraits placentaires, l'acide kojique, l'acide férulique, l'arbutine, des dérivés de dihydroxybenzène (WO 00/47045), des dérivés du guaiacol (WO 00/47179), le 4-(2,3-dihydroxyphényl)-cyclohexanol (WO 00/56279), des dérivés du résorcinol (WO 00/56702), des amides phénoliques (WO 99/32077). Ces substances peuvent présenter certains inconvénients. Elles peuvent être instables, nécessiter une utilisation à des concentrations élevées, manquer de spécificité quant à leur mode d'action, ou présenter un pouvoir cytotoxique ou irritant.

L'utilisation topique de substances dépigmentantes efficaces et inoffensives est particulièrement recherchée en cosmétique et en dermatologie. On utilise notamment ces substances pour traiter des hyperpigmentations régionales par hyper-activité mélanocytaire telles que les mélasmas idiopathiques, les hyperpigmentations localisées par hyper-activité et prolifération mélanocytaire bénigne telles que les taches pigmentaires dites de senescence (lentigos séniles), les hyperpigmentations accidentelles telles que la photosensibilisation ou l'hyperpigmentation cicatricielle, ainsi que certaines leucodermies telles que le vitiligo. Dans ces derniers cas, à défaut de pouvoir repigmenter la peau, on atténue la pigmentation de la périphérie des zones dépigmentées pour donner à la peau une couleur plus homogène.

Des substances dépigmentantes sont également utilisées en tant qu'agents de blanchiment de la peau par certaines personnes, en particulier celles désignées plus haut, qui sont très réactives aux UV, pour éclaircir leur teint, notamment celui de leur visage et de leurs mains, afin de conserver une couleur de peau la plus claire ou la plus homogène possible, ou tout au moins de réduire les effets pigmentants des rayons UV.

Le problème posé aux professionnels est donc la conception, la fabrication ou l'isolement de nouvelles substances dépigmentantes ou de nouveaux agents blanchissant de la peau humaine, des poils ou des cheveux ne présentant pas les inconvénients des substances connues, c'est-à-dire qui soient non irritants, non toxiques et/ou non allergisants pour la peau et stables dans une composition.

L'utilisation d'un oligonucléotide antisens pour traiter les maladies dues à un dysfonctionnement des mélanocytes, en particulier le vitiligo et d'autres maladies dépigmentantes, a été décrite dans WO 99/25819. Dans ces pathologies cutanées, l'hypopigmentation résulte d'une teneur anormalement élévée en ténascine. Les oligonucléotides décrits dans ce document agissent contre l'hypopigmentation en régulant l'expression de la ténascine.

Au contraire, l'objet de la présente invention consiste à fournir un agent dépigmentant agissant sur le processus de la mélanogénèse, destiné d'une part, dans le cas d'une pigmentation sensiblement homogène, au blanchiment de la peau, des poils ou des cheveux, c'est-à-dire à diminuer leur pigmentation, et d'autre part, à lutter contre l'hyperpigmentation cutanée à savoir lorsque la peau présente une hétérogénéité de pigmentation.

Les inventeurs de la présente invention ont trouvé que des oligonucléotides peuvent s'hybrider avec les gènes ou les produits des gènes (tels que les ARN) codant pour la tyrosinase.

Ainsi, les oligonucléotides selon l'invention, en modulant l'expression de la tyrosinase dans les mélanocytes, interviennent en amont de la réaction de la mélanogénèse présentée plus haut. Cette activité existe même à très faible concentration, ce qui augmente l'intérêt de ces oligonucléotides. De plus, les oligonucléotides selon l'invention ne présentent aucune cytotoxicité.

Les oligonucléotides selon l'invention offrent une solution idéale aux problèmes posés par les substances utilisées classiquement. Les substances connues qui inhibent l'activité de la tyrosinase (notamment l'hydroquinone et ses dérivés, l'acide ascorbique et ses dérivés, des extraits placentaires, l'acide kojique, l'arbutine) présentent de multiples effets secondaires inacceptables du fait de leur faible spécificité. La présente invention résout donc les problèmes rencontrés par les chercheurs antérieurs en modulant la production d'enzymes essentielles de la pigmentation au lieu d'inhiber directement les enzymes pour obtenir l'effet dépigmentant.

Les enzymes essentielles fonctionnant de concert sous la forme d'un complexe, les inventeurs on décrit de nouveaux oligonucléotides qui inhibent l'expression de l'une ou de l'autre des enzymes du complexe afin de résoudre le problème posé.

Les oligonucléotides selon l'invention sont nouveaux en tant que tels et nouveaux en tant que médicaments.

La présente invention concerne un oligonucléotide comportant un nombre de nucléotides compris entre 18 et 25, et de préférence encore comportant un nombre égal à 20, capable de s'hybrider avec le gène ou un produit du gène codant pour la tyrosinase, l'oligonucleolide inhibe l'expression de la tyrosinase et présente au moins 80% d'identité avec l'une des séquences SEQ ID NO.6 à SEQ ID NO.10 ayant la signification suivante :
- SEQ ID NO.6 5'-AGGAACTGGCTAATTGGAGT-3'
- SEQ ID NO.7 5'-CAAGGTCTGCAGGAACTGGC-3'
- SEQ ID NO.8 5'-CCTCACAAGGTCTGCAGGAA-3'
- SEQ ID NO.9 5'-CTACAGACAATCTGCCAAGA-3'
- SEQ ID NO.10 5'-GCATTCTTCCTCTAGTCCTC-3'

La présente invention a également pour objet un oligonucléotide en tant que produit nouveau dont la séquence est l'une des séquences SEQ ID NO.6 à NO.10 décrites ci-dessus.

Dans le cadre de la présente invention, on entend par « gène codant pour la tyrosinase », la séquence génomique du gène de la tyrosinase. De même, on entend par « gène codant pour la TRP-1 », la séquence génomique du gène de la TRP-1.

Les rôles clés de la tyrosinase et de la TRP-1 dans la mélanogénèse sont connus. L'utilisation d'oligonucléotides dirigés contre un ARN messager codant pour une enzyme ou une protéine afin d'en moduler l'expression est également connue. Cependant, la technique élaborée par les inventeurs de la présente invention n'avait jamais été utilisée comme moyen de dépigmentation.

Les oligonucléotides selon l'invention sont déterminés pour s'hybrider directement à l'ARN messager ou au gène. Ils permettent ainsi de réaliser une modulation ultime de la quantité de tyrosinase produite par les gènes.

Dans le présent document, le terme "hybridation" est utilisé pour désigner la formation de liaisons hydrogènes, aussi connue comme appariement Watson-Crick entre les bases complémentaires, usuellement sur deux brins d'acide nucléique pour former un duplex en double hélice.

Le degré de complémentarité entre deux séquences d'acide nucléique de longueur identique est déterminé en comparant, après alignement, la première séquence avec la séquence complémentaire de la seconde séquence. Le degré de complémentarité est calculé en déterminant le nombre de positions identiques pour lesquelles le nucléotide est identique entre les deux séquences ainsi comparées, en divisant ce nombre de positions identiques par le nombre total de positions et en multipliant le résultat obtenu par 100 pour obtenir le degré de complémentarité entre ces deux séquences.

Le terme "hybridation spécifique" signifie en particulier qu'il existe un degré de complémentarité suffisant pour éviter la fixation non spécifique de l'oligonucléotide sur une séquence non ciblée dans les conditions où la fixation spécifique est souhaitée. II est entendu que l'oligonucléotide n'a pas besoin de présenter une complémentarité de 100% avec la séquence de l'acide nucléique cible pour s'hybrider spécifiquement. En particulier, un oligonucléotide présentant un degré de complémentarité au moins égal à 80 % environ est capable de s'hybrider spécifiquement avec l'acide nucléique choisi comme cible.

Les oligonucléotides selon l'invention s'hybrident spécifiquement avec le gène ou le produit du gène codant..pour la tyrosinase. En particulier, les oligonucléotides de l'invention sont capables de s'hybrider soit avec l'ADN du gène qui code pour la tyrosinase, soit encore avec l'ARNm dérivant de ce gène. Les oligonucléotides selon l'invention comportent un nombre de nucléotides suffisant en identité et en nombre pour s'hybrider de façon spécifique. Cette propriété est couramment appelée "antisens".

Dans le cadre de la présente invention, on entend par « ADN qui codent pour la tyrosinase » aussi bien les exons que les introns, en particulier les exons.

Il est précisé que :
◆ les oligonucléotides de séquences SEQ ID NO 1, SEQ ID NO 2, SEQ ID NO 3 et SEQ ID NO 4, s'hybrident spécifiquement avec la région 5' non codante du gène ou de l'ARNm codant pour la TRP-1,
◆ l'oligonucléotide de séquence SEQ ID NO 5, s'hybride spécifiquement avec la région présentant le codon d'initiation du gène ou de l'ARNm codant pour la TRP-1,
◆ les oligonucléotides de l'invention de séquences SEQ ID NO 6, SEQ ID NO 7 et SEQ ID NO 8, s'hybrident spécifiquement avec la région 5' non codante du gène ou de l'ARNm codant pour la tyrosinase,
◆ l'oligonucléotide de l'invention de séquence SEQ ID NO 9, s'hybride spécifiquement avec la région codante du gène ou de l'ARNm codant pour la tyrosinase,
◆ l'oligonucléotide de l'invention de séquence SEQ ID NO 10, s'hybride spécifiquement avec la région présentant le codon d'initiation du gène ou de l'ARNm codant pour la tyrosinase,
◆ l'oligonucléotide de séquence SEQ ID NO 11, s'hybride spécifiquement avec la région 5' non codante du gène ou de l'ARNm codant pour la TRP-1.

La présente invention a aussi pour objet des oligonucléotides, comprenant une ou plusieurs modifications chimiques au niveau de leurs parties sucres, leurs parties nucléobases ou leur squelette internucléotidique qui confèrent des caractéristiques physico-chimiques souhaitables aux oligonucléotides selon l'invention telles qu'une biodisponibilité accrue, l'augmentation de l'affinité pour les séquences cibles, l'augmentation de l'internalisation cellulaire ou une meilleure stabilité biologique ou l'augmentation de la stabilité en présence de nucléases cellulaires.

A titre d'exemple, les modifications pouvant conférer ces caractéristiques sont les dérivés 2'-O-alkyle et 2'-O-fluoro sur la partie sucre du nucléoside, et les dérivés phosphorothioates ou les dérivés méthylphosphonates au niveau du squelette internucléotidique.

Dans le présent document, le terme "oligonucléotide" se réfère à des polynucléotides formés à partir de nucléobases naturelles et de groupements pentafuranosyles (sucre) formant des nucléosides qui sont reliés entre eux par liaisons phosphodiesters natives. Le terme "oligonucléotides" se réfère donc aux espèces naturelles ou aux espèces synthétiques formés à partir de sous unités naturelles ou de leurs homologues proches.

Le terme "oligonucléotides" peut se référer aussi aux parties qui ont des fonctions similaires aux oligonucléotides naturels mais qui peuvent présenter des portions non naturelles. Les oligonucléotides peuvent avoir des parties sucres, des parties nucléobases ou des liaisons intemucléotidiques modifiées. Parmi les modifications possibles, les modifications préférées sont les dérivés 2'-O-alkyle sur la partie sucre, en particulier les dérivés 2'-O-éthyloxyméthyle ou 2'-O-méthyle, ou les phosphorothioates ou les méthylphosphonates pour le squelette internucléotidique.

Les oligonucléotides chimériques sont compris dans les modifications préférentielles de l'invention. Les oligonucléotides contiennent au moins deux régions chimiquement différentes, chacune comprenant au moins un nucléotide. Il s'agit en particulier d'une ou de plusieurs régions comprenant un nucléotide modifié qui confère une ou plusieurs propriétés bénéfiques comme par exemple une meilleure stabilité biologique, une biodisponiblité accrue, l'augmentation de l'internalisation cellulaire ou l'augmentation de l'affinité pour l'ARN cible.

De façon préférée, le squelette internucléotidique peut être en tout ou partie phosphodiesters ou phosphorothioates ou méthylphosphonates, ou les combinaisons de liaisons phosphodiesters et/ou phosphorothioates et/ou méthylphosphonates.

Ainsi, l'oligonucléotide selon l'invention est caractérisé en ce qu'une partie des groupements phosphodiesters de son squelette internucléotidique est remplacée par des groupements phophorothioates et/ou des groupements méthylphosphonates.

Alternativement, l'oligonucléotide selon l'invention est caractérisé en ce que tous les groupements phophodiesters sont remplacés par des groupements phosphorothioates ou par des groupements méthylphosphonates.

Alternativement, les groupements phophodiesters sont remplacés en tout ou partie par des groupements phosphorothioates et/ou par des groupements méthylphosphonates.

Le terme "oligonucléotides" peut également se référer à des oligonucléotides auxquels on a greffé un vecteur d'administration circulaire de type plasmidique ou un vecteur d'administration linéaire de type acide nucléique ou peptidique.

Les oligonucléotides de l'invention sont nouveaux en tant que médicaments.

La présente invention a aussi pour objet une composition cosmétique ou dermatologique contenant au moins un oligonucléotide décrit précédemment et un milieu cosmétiquement ou dermatologiquement acceptable. Une telle composition peut contenir en outre, un ou plusieurs actifs visant à renforcer les effets recherchés.

La présente invention a également pour objet l'utilisation d'au moins une séquence oligonucléotidique dirigée contre un produit de transcription du gène codant pour la tyrosinase dans ou pour la fabrication d'une composition cosmétique ou dermatologique pour dépigmenter ou blanchir la peau, les poils ou les cheveux humains, ou enlever ou atténuer les taches pigmentaires de la peau humaine.

La présente invention se rapporte également à l'utilisation d'un oligonucléotide capable de s'hybrider spécifiquement avec le gène ou un produit du gène codant pour la tyrosinase, comme agent cosmétique, notamment pour dépigmenter ou blanchir la peau, les poils ou les cheveux humains, et pour atténuer les taches pigmentaires de la peau humaine.

La présente invention se rapporte également à l'utilisation d'au moins un oligonucléotide décrit précédemment pour la fabrication d'un médicament destiné au traitement ou à la prévention des maladies se traduisant par la surexpression de la tyrosinase par voie topique. Ce médicament peut être destiné à inhiber la synthèse de mélanine, notamment à dépigmenter ou à blanchir la peau, mais également pour le traitement ou la prévention des hyperpigmentations régionales par hyper-activité mélanocytaire telles que les mélasmas idiopathiques, des hyperpigmentations localisées par hyper-activité et prolifération mélanocytaire bénigne telles que les taches pigmentaires de sénescence (lentigos séniles), des hyperpigmentations accidentelles telles que la photosensibilisation ou la cicatrisation post-lésionnelle, et pour le traitement de certaines leucodermies telles que le vitiligo.

La présente invention a aussi pour objet l'utilisation d'au moins une séquence oligonucléotide dirigée contre un produit de transcription du gène codant pour la tyrosinase dans une composition cosmétique dépigmentante ou blanchissante de la peau humaine.

La présente invention a aussi pour objet une composition dépigmentante caractérisée en ce qu'elle contient, dans un milieu cosmétiquement ou dermatologiquement acceptable, au moins une séquence oligonucléotidique dirigée contre un produit de transcription du gène codant pour la tyrosinase

Selon un mode de mise en oeuvre préféré de la présente invention pour les compositions, les utilisations et le procédé de traitement précités, l'oligonucléotide est celui dont la séquence est définie ci-dessous:
- SEQ ID NO.6 5'-AGGAACTGGCTAATTGGAGT-3'

La composition cosmétique ou dermatologique selon l'invention est appropriée pour une utilisation topique et contient donc un milieu cosmétiquement ou dermatologiquement acceptable, c'est-à-dire compatible avec la peau. La séquence oligonucléotidique selon l'invention peut être présente en quantité allant de 0,00001% à 10% et de préférence de 0,0003% à 3% du poids total de la composition.

La composition de l'invention peut se présenter sous toutes les formes galéniques habituellement utilisées pour une application topique notamment sous forme d'une solution aqueuse, hydroalcoolique ou huileuse, d'une émulsion huile dans eau ou eau dans huile ou multiple, d'un gel aqueux ou huileux, d'un produit anhydre liquide, pâteux ou solide, d'une dispersion de particules polymériques, telles que des nanosphères et des nanocapsules, dans une phase aqueuse ou huileuse, ou encore d'une dispersion de vésicules lipidiques de type ionique ou non-ionique telle que décrite dans le brevet US 4,508,703.

Cette composition peut être plus ou moins fluide et avoir l'aspect d'une crème blanche ou colorée, d'une pommade, d'un lait, d'un gel, d'une lotion, d'un sérum, d'une pâte ou d'une mousse. Elle peut éventuellement être appliquée sur la peau sous forme d'aérosol. Elle peut également se présenter sous forme solide pulvérulent ou non, par exemple sous forme stick ou d'une poudre pressée. Elle peut encore se présenter sous la forme de patchs, de crayons, de pinceaux et d'applicateurs autorisant une application localisée sur les taches du visage ou des mains. Elle peut être utilisée comme produit de soin ou comme produit de maquillage.

De façon connue, la composition de l'invention peut contenir également les adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans les domaines considérés. Ces adjuvants, selon leur nature, peuvent être introduits dans la phase grasse, dans la phase aqueuse, dans les vésicules lipidiques ou dans les nanoparticules.

Lorsque la composition cosmétique ou dermatologique de l'invention est une émulsion, la proportion de la phase grasse peut aller en général de 5 à 80% en poids, et de préférence de 5 à 50% en poids par rapport au poids total de la composition. Les huiles, les émulsionnants et les co-émulsionnants utilisés dans la composition sous forme d'émulsion sont choisis parmi ceux classiquement utilisés dans le domaine considéré. L'émulsionnant et le co-émulsionnant sont présents, dans la composition, en une proportion allant généralement de 0,3% à 30% en poids, et de préférence de 0,5% à 20% en poids par rapport au poids total de la composition.

Comme huiles utilisables en association avec les oligonucléotides selon l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matière grasses des alcools gras (alcool cétylique), des acides gras, des cires (cire de Carnauba, ozokérite).

Comme émulsionnants et coémulsionnants utilisables en association avec les oligonucléotides selon l'invention, on peut citer par exemple les esters d'acide gras et de polyéthylène glycol tels que le stéarate de PEG-20 et les esters d'acide gras et de glycérine tels que le stéarate de glycéryle.

Comme gélifiants hydrophiles utilisables en association avec les oligonucléotides selon l'invention, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles. Comme gélifiants lipophiles, on peut citer les argiles modifiées comme les bentones, les sels métalliques d'acides gras, la silice hydrophobe et les polyéthylènes.

La présente invention a pour objet une composition cosmétique ou dermatologique contenant au moins un oligonucléotide décrit précédemment et un ou plusieurs autres agents actifs.

La présente invention concerne également l'utilisation d'au moins un oligonucléotide tel que décrit précédemment pour la fabrication d'un médicament destiné à être administré de façon simultanée, séparée ou étalée dans le temps en association avec un ou plusieurs autres actifs.

Lesdits actifs utilisables en association avec les oligonucléotides selon l'invention, utilisés purs ou provenant d'extraits renfermant ces molécules, sont notamment les composés suivants: l'acide ellagique et ses dérivés ; l'hydroquinone ; l'arbutine ; le résorcinol et ses dérivés ; la vitamine C et ses dérivés ; le pantothénate sulfonate et ses dérivés ; l'acide kojique ; les extraits placentaires ; des molécules interférant directement ou indirectement avec l'alpha-mélanocyte stimulating hormone (α-MSH) ou son récepteur ou l'hormone adrénocorticotropique (ACTH) ; les polyols tels que la glycérine, le glycol ou le propylène glycol ; les vitamines ; les agents kératolytiques ou desquamants tels que l'acide salicylique et ses dérivés ; les alpha-hydroxyacides tels que l'acide lactique ou l'acide malique, seuls ou greffés ; l'acide ascorbique et ses dérivés ; l'acide rétinoïque ; le rétinaldéhyde ; le rétinol et ses dérivés tels que le palmitate, le propionate ou l'acetate, en préparation liposomique ou non ; des agents antiglycations ou antioxydants pris seuls ou en association tels que le tocophérol et ses dérivés, la thiotaurine, l'hypotaurine, l'aminoguanidine, la thiamine pyrophosphate, la pyridoxamine, la lysine, l'histidine, l'arginine, la phénylalanine, la pyridoxine, l'adénosine triphosphate ; les agents anti-inflammatoires tels que le stéaryl glycyrrhétinate ; les agents apaisants et leurs mélanges, les filtres solaires chimiques ou physiques tels que le méthoxycinnamate d'octyle, le butyl-méthoxydibenzoyl-méthane, l'oxyde de titane et l'oxyde de zinc micronisés ; et les acides désoxyribonucléiques ou nucléiques. En cas d'incompatibilité, ces autres actifs et/ou les oligonucléotides de l'invention peuvent être incorporés dans des sphérules, notamment des vésicules formés de lipides amphiphiles ioniques ou non ioniques telles que décrites dans le brevet US 4,508,703, ou des nanoparticules.

Les exemples suivants illustrent la présente invention sans toutefois la limiter.

Pour des raisons de stabilité dans les milieux de culture in-vitro et conformément à l'usage, les exemples 2 à 4 ont été réalisés avec des dérivés phosphorothioates et les exemples 5 à 12 ont été réalisés indifféremment avec des dérivés phosphorothioates ou phosphodiesters.

Dans les exemples qui suivent, tous les pourcentages sont donnés en poids, sauf indications contraires.

### Exemple 1 : Synthèse des Oligonucléotides

A titre d'exemples, des oligonucléotides ont été synthétisés avec un synthétiseur automatique (Perseptive Biosystems Expedite modèle 8909) en utilisant la chimie standard des dérivés phosphoramidites en utilisant les protocoles du constructeur. Les β-cyanoéthyldiisopropylphosphoramidites ont été fournis par la société Perseptive Biosystems. Pour les oligonucléotides phosphodiesters, l'étape d'oxydation du phosphite a été effectuée avec une solution d'iode. En ce qui concerne les oligonucléotides phosphorothioates, l'étape d'oxydation du phosphite a été effectuée en utilisant une solution 0,05 M de 3H-1,2-benzodithiol-3-one 1,1-dioxide dans de l'acétonitrile anhydre. Après clivage de la colonne (Controlled Pore Glass, Perseptive Biosystems) et déprotection totale de la séquence par un traitement de 18h à 55°C par une solution d'ammoniaque à 33%, les oligonucléotides ont été purifiées par précipitation dans l'éthanol en présence d'acétate de sodium. Des contrôles par chromatographie liquide haute pression ont été ensuite réalisés par chromatographie échangeuses d'ions avec élution par un gradient de chlorure de sodium et par chromatographie en phase inverse C18 avec élution par un gradient d'acétonitrile en présence d'acétate de triéthylammonium.

Les oligonucléotides synthétisés sont décrits dans le tableau 1. Il s'agit des 11 premières séquences de ce tableau, numérotées de SEQ ID NO.1 à SEQ ID NO.11. Leur activité dépigmentante a fait l'objet d'études rapportées dans les exemples suivants.

Dans le tableau 1, les numéros mentionnés sous chaque extrémité des séquences indiquent la position de l'oligonucléotide dans les séquences d'origine.

Les séquences proviennent respectivement de la séquence dite « HUMTYRA » de l'ADNc de la tyrosinase humaine, publiée par Shibahara et al., Tohoku J. Exp. Med. 156 : 403 (1988) (Genbank accession number M 27160), de la séquence dite « HSTYRRP » de l'ADNc de la TRP-1 humaine, publiée par Cohen et al., Nucl. Acides Res. 18 : 2807 (1990) (Genbank accession number X 51420), et d'une autre séquence de la TRP-1, dite « AF001295 » publiée par Box et al., Mamm. Genome 9 : 50 (1998) (Genbank accession number AF 001295).

Par ailleurs, à titre comparatif pour confirmer la spécificité des oligonucléotides selon l'invention vis à vis des gènes ou des produits de gènes codant pour la tyrosinase ou la TRP-1, deux oligonucléotides basés sur la séquence SEQ ID N°2 de l'invention, ont été synthétisés, à savoir :
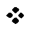 Une séquence dite « contrôle sens », désignée SEQ ID N°12 dans le tableau 1, consistant à inverser l'ordre des bases de la séquence SEQ ID N°2.
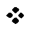 Une séquence dite « contrôle brouillé », désignée SEQ ID N°13, également dans le tableau 1, comprenant les mêmes bases, en nature et en nombre, que celles de la séquence SEQ ID N°2, mais placées dans un ordre quelconque.

**TABLEAU 1**

| SEQ ID NO. | SEQUENCE OLIGONUCLEOTIDE | LOCUS |
|---|---|---|
| 1. | 5'-GCAAAACAAAGACCTGGTTT-3' 111 92 | HSTYRRP |
| 2. | 5'-AGACCTGGTTTGCAGCTCTT-3' 102 83 | HSTYRRP |
| 3. | 5'-TGCTTGAAATAAGAGTGCAA-3' 127 108 | HSTYRRP |
| 4. | 5'-AAAATCCAGCTCACAATCCT-3' 53 34 | HSTYRRP |
| 5. | 5'-AGGAGCACTCATTCTGCTTG-3' 141 122 | HSTYRRP |
| 6. | 5'-AGGAACTGGCTAATTGGAGT-3' 475 457 | HUMTYRA |
| 7. | 5'-CAAGGTCTGCAGGAACTGGC-3' 485 466 | HUMTYRA |
| 8. | 5'-CCTCACAAGGTCTGCAGGAA-3' 490 471 | HUMTYRA |
| 9. | 5'-CTACAGACAATCTGCCAAGA-3' 1332 1313 | HUMTYRA |
| 10 | 5'-GCATTCTTCCTCTAGTCCTC-3' 506 487 | HUMTYRA |
| 11. | 5'-TTCCAGTACCTCACAATCCT-3' 5875 5856 | AF001295 |
| 12. | 5'-TTCTCGACGTTTGGTCCAGA-3' | CONTROLE SENS SEQ ID NO.2 |
| 13. | 5'-ACGTTTCTCGCCTAGTGATG-3' | CONTROLE BROUILLE SEQ ID NO.2 |

### Exemple 2 : Effet dépigmentant des oligonucléotides selon l'invention sur des mélanocytes humains normaux (MHN)

Les oligonucléotides selon l'invention figurant dans le tableau 1 de l'exemple 1 (SEQ ID N° 1 à SEQ ID N° 11) sont étudiés pour leur action sur la mélanogénèse, et donc sur leur capacité à moduler la production de pigments mélaniques par les mélanocytes. A titre d'éléments de comparaison, les oligonucléotides « contrôle sens » (SEQ ID N° 12) et « contrôle brouillé » (SEQ ID N° 13) sont également étudiés pour cette action.

Il est rappelé qu'au cours du mécanisme réactionnel de la mélanogénèse, la tyrosinase et la TRP-1 forment un complexe enzymatique qui catalyse la transformation de la L-dopa en dopaquinone puis en dopachrome. Ces deux enzymes agissent de concert et il semble qu'elles ne fonctionnent jamais l'une sans l'autre. En effet, lorsqu'une cellule est déplétée en TRP-1 par l'utilisation d'oligonucléotides antisens dirigés contre TRP-1, une diminution de l'activité tyrosinase encore appelée « dopa-oxydase » est observée. La tyrosinase est également dénommée « dopa-oxydase » en raison de sa nature multi-fonctionnelle ; elle remplit en effet la même fonction que la dopa-oxydase au cours de l'oxydation de la tyrosine en dopa.

Le principe du présent test est basé sur la mesure de la vitesse de réaction de la dopa-oxydase dans la transformation de la L-dopa, utilisée comme substrat, en dopachrome. L'apparition de dopachrome est quantifiée par mesure de densité optique à intervalles de temps déterminés, ce qui permet de calculer la vitesse de réaction de la dopa-oxydase pour chacun des oligonucléotides testés et pour l'essai témoin. A partir des résultats obtenus, il sera donc possible d'apprécier l'action des oligonucléotides testés sur la mélanogénèse. Une baisse de la vitesse de réaction par rapport à l'essai témoin correspondra à une diminution de l'activité enzymatique, et par conséquent, à la réduction de la formation de pigments mélaniques, et donc à un effet dépigmentant.

### Préparation de cultures de mélanocytes et traitements par les oligonucléotides :

Les mélanocytes sont obtenus à partir de peau de prépuce d'un enfant de type caucasien. Les fragments de peau sont rincés au PBS (Gibco, Paisley, GB), puis à l'éthanol à 70%. Après un dernier lavage au PBS, la peau est découpée en fines bandelettes de 1 mm comportant un minimum de derme. Le derme et l'épiderme sont dissociés par incubation des bandelettes dans une solution de trypsine à 0,25% (Gibco, Paisley, GB) pendant une nuit à 4°C. Après incubation, les cellules épidermiques sont récupérées en grattant les cellules au scalpel. L'action de la trypsine est stoppée en plaçant les cellules dans un milieu E-199 (Gibco, Paisley, GB) contenant 10% (VN) de sérum de veau foetal (Gibco, Paisley, GB). Après homogénéisation et élimination des cornéocytes flottant en surface, la suspension cellulaire est filtrée, centrifugée et le culot est repris dans un milieu d'adhérence (Milieu 1) dont la composition est présentée dans le tableau suivant, dans lequel EGF désigne un facteur de croissance épidermique.

| **COMPOSITION DU MILIEU 1** | | |
|---|---|---|
| Composés | Fournisseur, référence | Concentration finale dans le milieu dans le milieu |
| - Milieu E-199 | Gibco, 31150-022 | qsp 100% (v/v) |
| - Sérum de veau foetal | Gibco, 10099-133 | 10 % (v/v) |
| - Hydrocortisone | Sigma, H-0135 | 0,4 µg/ml |
| - L-glutamine | Gibco, 25030-024 | 2 mM |
| - EGF | Sigma, E-4127 | 10 ng/ml |

Après dénombrement de la population cellulaire, les cellules sont ensemencées en flasques à raison de 200000/cm². Les cultures sont maintenues à 37°C, en atmosphère saturée en humidité et avec 5% CO2. Après adhésion des cellules, le milieu d'adhérence est remplacé par du milieu KSFM (Gibco, Paisley, GB), qui sera renouvelé toutes les 48h.

Les cellules épidermiques ainsi mises en culture vont permettre l'obtention d'une coculture kératinocytes-mélanocytes. Dès que cette coculture est à environ 70% de confluence, le milieu KSFM est remplacé par un milieu sélectif (Milieu 2) favorisant la croissance des mélanocytes, et dont la composition figure au tableau ci-après, dans lequel IBMX désigne la 3-isobutyl-1-méthyl-xanthine, et PMA désigne le phorbol-12-myristate-13-acétate.

| **COMPOSITION DU MILIEU 2** | | |
|---|---|---|
| Composés | Fournisseur, référence | Concentration finale dans le milieu |
| - Milieu E-199 | Gibco, 31150-022 | qsp 100% (vlv) |
| - Sérum de veau foetal | Gibco, 10099-133 | 10 % (v/v) |
| - L-glutamine | Gibco, 25030-024 | 0,4 µg/ml |
| - IBMX | Sigma, I-5879 | 2 mM |
| - PMA | Sigma, P-8139 | 10 ng/ml |
| - Généticine | Gibco, 066-01811Y | 100µg/ml |

48 à 72 h après, les cellules sont décollées de la flasque à l'aide d'un mélange trypsine 0,1%-EDTA 0,05% (Gibco, Paisley, GB) pendant 1 à 2 minutes à température ambiante. La suspension cellulaire obtenue est centrifugée et remise en milieu sélectif. Les cellules sont à nouveau traitées 24 à 48h plus tard avec le mélange trypsine-EDTA (0,1%-0,05%), pendant 1 à 2 minutes à température ambiante, puis réensemencées dans un milieu de prolifération spécifique des mélanocytes (Milieu 3).

Le Milieu 3 est composé de 10 % de Milieu 2 et de 90 % de Milieu A. Le Milieu A est le milieu kératinocyte SFM (Gibco, 17005-034).

A ce stade, la population cellulaire obtenue est pure, constituée exclusivement de mélanocytes humains normaux (MHN).

Avant l'étude, les MHN sont placés pendant une semaine dans un milieu ne contenant aucun activateur métabolique puissant tels les esters de phorbol ou l'IBMX (Milieu 4).

Le Milieu 4 est composé de 10 % de Milieu B et 90 % de Milieu A décrit précédemment. La composition du milieu B est décrite dans le tableau ci-dessous.

| **COMPOSITION DU MILIEU B** | | |
|---|---|---|
| Composés | Fournisseur, référence | Concentration finale dans le milieu |
| - Milieu E-199 | Gibco, 31150-022 | qsp 100% (v/v) |
| - Sérum de veau foetal | Gibco, 10099-133 | 10 % (v/v) |
| - L-glutamine | Gibco, 25030-024 | 2 mM |

Les MHN sont ensemencés en microplaques 96 puits (Falcon, Franklin Lakes, NJ, USA), à raison de 10000 cellules par puits, dans 200 µl de milieu 4.

Les oligonucléotides à tester sont préparés extemporanément dans le milieu d'étude, respectivement à des concentrations de 10 nM, 100 nM, 250 nM, 500 nM, 1µM. Les traitements avec les différents oligonucléotides sont réalisés chaque jour pendant 7 jours. Pour chaque concentration, 3 essais sont réalisés.

### Mesure de vitesses de réaction de l'activité dopa-oxydase :

A la fin de ces traitements répartis sur 7 jours, des mesures de l'activité tyrosinase ou « dopa-oxydase » sont réalisées.

Les cellules sont rincées au PBS, puis 50 µl de tampon de lyse (Triton 100X Sigma, T-9284) à 0,5 % dans du PBS (Gibco, 14190-094)) sont ajoutés dans les puits et la plaque est mise sous agitation pendant une heure à 4°C. La réaction est initiée en ajoutant 50 µl de substrat (L-DOPA 10 mM, Sigma) dans chaque puits. L'apparition du dopachrome est mesurée toutes les 2 minutes à 450 nm pendant une heure et à 37°C, sous agitation régulière, au moyen d'un lecteur de densité optique, lecteur de microplaques 340 ATTC (SLT-Labinstrument, Grödig/Salzburg, Autriche). Les vitesses de réaction sont calculées et exprimées en 10⁻⁴ Unité DO/mn.

Les résultats obtenus pour les cultures traitées par les oligonucléotides et pour les cultures témoin (non traitées) figurent dans le tableau 2 (σ : écart-type).

**TABLEAU 2**

| Numéros d'identification et concentrations des oligonucléotides testés | | Vitesses de réaction 10⁻⁴ unité DO/mn | Vitesses de réaction (témoin sans oligonucléotide) | % de variation par rapport au témoin |
|---|---|---|---|---|
| SEQ ID NO.1 | 10 nM | 21,8 σ = 1,0 | 26,4 σ = 1,8 | -17,4 |
| | 100 nM | 23,3 σ = 0,4 | 26,4 σ = 1,8 | -11,7 |
| | 250 nM | 20,3 σ = 0,3 | 26,4 σ = 1,8 | -23,1 |
| | 500 nM | 19,5 σ = 2,9 | 26,4 σ = 1,8 | -26,1 |
| | 1 µM | 23,9 σ = 1,4 | 26,4 σ = 1,8 | -9,4 |
| SEQ ID NO.2 | 10 nM | 20,5 σ = 1,7 | 26,4 σ = 1,8 | -22,3 |
| | 100 nM | 18,8 σ = 1,5 | 26,4 σ = 1,8 | -28,8 |
| | 250 nM | 19,2 σ = 0,7 | 26,4 σ = 1,8 | -27,3 |
| | 500 nM | 19,7 σ = 1,7 | 26,4 σ = 1,8 | -25,4 |
| | 1 µM | 22,9 σ = 1,1 | 26,4 σ = 1,8 | -13,3 |
| SEQ ID NO.3 | 10 nM | 23,7 σ = 3,1 | 26,4 σ = 1,8 | -10,2 |
| | 100 nM | 20 σ = 0,7 | 26,4 σ = 1,8 | -24,2 |
| | 250 nM | 21,1 σ = 0,6 | 26,4 σ = 1,8 | -20,1 |
| | 500 nM | 19,9 σ = 1,7 | 26,4 σ = 1,8 | -24,6 |
| | 1 µM | 23,6 σ = 0,6 | 26,4 σ = 1,8 | -10,6 |
| SEQ ID NO.4 | 10 nM | 22,9 σ = 1,2 | 26,4 σ = 1,8 | -13,3 |
| | 100 nM | 19,9 σ = 1,6 | 26,4 σ = 1,8 | -24,6 |
| | 250 nM | 20,9 σ = 1,4 | 26,4 σ = 1,8 | -20,8 |
| | 500 nM | 21,2 σ = 1,7 | 26,4 σ = 1,8 | -19,7 |
| | 1 µM | 20 σ = 1,2 | 26,4 σ = 1,8 | -24,2 |
| SEQ ID NO.5 | 10 nM | 23,5 σ = 0,7 | 26,4 σ = 1,8 | -10,9 |
| | 100 nM | 19,3 σ = 0,9 | 26,4 σ = 1,8 | -26,9 |
| | 250 nM | 17,9 σ = 1,7 | 26,4 σ = 1,8 | -32,2 |
| | 500 nM | 20,5 σ = 0,6 | 26,4 σ = 1,8 | -22,4 |
| | 1 µM | 21,3 σ = 0,8 | 26,4 σ = 1,8 | -19,3 |
| SEQ ID NO.6 | 10 nM | 20,7 σ = 1,8 | 26,4 σ = 1,8 | -21,6 |
| | 100 nM | 22,0 σ = 0,6 | 26,4 σ = 1,8 | -16,7 |
| | 250 nM | 20,1 σ = 2,5 | 26,4 σ = 1,8 | -23,9 |
| | 500 nM | 18,7 σ = 2,6 | 26,4 σ = 1,8 | -29,2 |
| | 1 µM | 20,5 σ = 1,1 | 26,4 σ = 1,8 | -22,4 |
| SEQ ID NO.7 | 10 nM | 26,6 σ = 2,2 | 25,0 σ = 0,9 | +6,4 |
| | 100 nM | 25,3 σ = 1,1 | 25,0 σ = 0,9 | +1,2 |
| | 250 nM | 23,8 σ = 1,3 | 25,0 σ = 0,9 | -4,8 |
| | 500 nM | 21,7 σ = 0,9 | 25,0 σ = 0,9 | -13,2 |
| | 1 µM | 22,6 σ = 0,7 | 25,0 σ = 0,9 | -9,6 |
| SEQ ID NO.8 | 10 nM | 24,1 σ = 0,1 | 25,0 σ = 0,9 | -3,6 |
| | 100 nM | 23,7 σ = 0,6 | 25,0 σ = 0,9 | -2,5 |
| | 250 nM | 25,1 σ = 1,0 | 25,0 σ = 0,9 | +0,4 |
| | 500 nM | 23,4 σ = 0,6 | 25,0 σ = 0,9 | -6,4 |
| | 1 µM | 23,7 σ = 0,2 | 25,0 σ = 0.9 | -5,2 |
| SEQ ID NO.9 | 10 nM | 27,1 σ = 0,7 | 25,0 σ = 0,9 | +8,4 |
| | 100 nM | 22,3 σ = 0,6 | 25,0 σ = 0,9 | -10,8 |
| | 250 nM | 22,6 σ = 0,2 | 25,0 σ = 0,9 | -9,6 |
| | 500 nM | 23,2 σ = 0,5 | 25,0 σ = 0,9 | -7,2 |
| | 1 µM | 25,0 σ = 0,7 | 25,0 σ = 0,9 | 0 |
| SEQ ID NO.10 | 10 nM | 26,4 σ = 0,9 | 25,0 σ = 0,9 | +5,6 |
| | 100 nM | 24,9 σ = 0,5 | 25,0 σ = 0,9 | -0,4 |
| | 250 nM | 23,1 σ = 0,3 | 25,0 σ = 0,9 | -7,6 |
| | 500 nM | 23,3 σ = 0,6 | 25,0 σ = 0,9 | -6,8 |
| | 1 µM | 26,9 σ = 0,7 | 25,0 σ = 0,9 | +7,6 |
| SEQ ID NO.11 | 10 nM | 25,7 σ = 0,9 | 25,0 σ = 0,9 | +2,8 |
| | 100 nM | 24,8 σ = 0,3 | 25,0 σ = 0,9 | -0,8 |
| | 250 nM | 25,8 σ = 0,7 | 25,0 σ = 0,9 | +3,2 |
| | 500 nM | 22,1 σ = 4,4 | 25,0 σ = 0,9 | -11,6 |
| | 1 µM | 23,9 σ = 0,8 | 25,0 σ = 0,9 | -4,4 |
| SEQ ID NO.12 | | | | |
| | 50 nM | 34,6 σ = 1,0 | 32,5 σ = 1,4 | + 6,5 |
| | 100 nM | 33,2 σ = 0,7 | 32,5 σ = 1,4 | + 2,2 |
| | 150 nM | 33,9 σ = 0,7 | 32,5 σ = 1,4 | + 4,3 |
| SEQ ID NO.13 | | | | |
| | 50 nM | 33,5 σ = 1,2 | 32,5 σ = 1,4 | + 3,1 |
| | 100 nM | 36,3 σ = 0,6 | 32,5 σ = 1,4 | + 11,7 |
| | 150 nM | 34,0 σ = 1,2 | 32,5 σ = 1,4 | + 4,6 |

Des résultats figurant au tableau 2, il ressort clairement que le traitement des cultures de mélanocytes par les oligonucléotides de l'invention (SEQ ID NO.1 à SEQ ID NO.11) conduit à une diminution de la vitesse de réaction de l'activité dopa-oxydase. Cette diminution est particulièrement nette pour les oligonucleotides SEQ ID NO.1 à SEQ ID NO.6.

En revanche pour les oligonucléotides testés à titre de comparaison par rapport à l'oligonucléotide SEQ ID NO.2, à savoir le « contrôle sens » SEQ ID NO.12 et le « contrôle brouillé » SEQ ID NO.13, on constate une augmentation de la vitesse de réaction de l'activité dopa-oxydase à chacune des concentrations testées. Par exemple à la concentration de 100 nM, la vitesse de réaction augmente de 2,2 % et de 11,7 % respectivement pour le « contrôle sens » et le « contrôle brouillé », tandis qu'elle diminue de 28,8 % dans le cas de l'oligonucléotide anti-sens SEQ ID NO.2 selon l'invention.

On peut déduire de cette dernière observation que l'activité des oligonucléotides de l'invention, qui diminuent la vitesse de réaction de l'activité dopa-oxydase, est due nécessairement à l'hybridation spécifique de l'oligonucléotide avec l'ARN messager cible codant pour la tyrosinase ou celui codant pour la TRP-1.

De ce fait, cette interaction oligonucléotide - ARNm va empêcher la traduction de l'information portée par l'ARN messager ciblé et donc entraîner une diminution de l'expression intracellulaire de la tyrosinase, ou de la TRP-1 selon la séquence antisens utilisée. Autrement dit, cette intéraction va diminuer la néosynthèse de la tyrosinase ou de la TRP-1. En raison du renouvellement naturel de ces enzymes, ceci entraîne une diminution de la quantité de tyrosinase et de TRP-1 dans les cellules, et par conséquent une diminution de l'activité de ces enzymes en rapport avec la synthèse de mélanine. De fait, les oligonucléotides de l'invention agissent en réduisant le renouvellement de la tyrosinase et de la TRP-1, au lieu d'intervenir sur le tyrosinase déjà présente dans les mélanocytes.

La conséquence de cette action au niveau des mélanocytes est la réduction de la capacité de synthèse de mélanines par ces cellules, d'où, selon les cas et les individus concernés, la production d'un effet blanchissant ou dépigmentant.

### Exemple 3 : Effet dépigmentant d'un oligonucléotide selon l'invention sur des mélanocytes humains normaux d'un donneur adulte mulâtre

Les mélanocytes utilisés ici sont obtenus à partir de peau de cuisses d'un donneur adulte mulâtre. Les fragments de peau sont rincés au PBS (Gibco, Paisley, GB) puis lavés avec de l'éthanol 70% (V/V) dilué à 50% dans du PBS. Après lavage au PBS, les fragments de peau sont grattés à l'aide d'un coupe-cors. Les morceaux obtenus sont grattés dans de la trypsine 0,05% (Difco Laboratories, West Molesy, GB) pendant une heure à 37°C afin de permettre leur dissociation. L'épiderme est alors recueilli dans du milieu E-199 (Gibco, Paisley, GB) contenant 10% de sérum de veau foetal (SVF, Gibco, Paisley, GB). Après homogénéisation et élimination des fragments de couche cornée flottant en surface, la suspension cellulaire est filtrée puis centrifugée. Le culot cellulaire obtenu est repris dans du Milieu 1 décrit à l'exemple 2. Les cellules sont dénombrées et une mesure de la viabilité cellulaire est réalisée sur cellule de Thoma par le test d'exclusion du bleu trypan. Les cellules épidermiques sont finalement ensemencées dans du Milieu 1, en flasques à raison de 80000 cellules/cm². Les cultures sont maintenues à 37°C, en atmosphère saturé en humidité et avec 5% de CO₂.

Le Milieu 1 est remplacé par un Milieu 6 dont la composition est précisée au tableau ci-dessous, dans lequel B-FGF désigne un facteur de croissance de fibroblastes de base, et PdBu désigne le phorbol-12,13-dibutyrate.

| **COMPOSITION DU MILIEU 6** | | |
|---|---|---|
| Composés | Fournisseur, référence | Concentration finale dans le milieu |
| - Milieu E-199 | Gibco, 31150-022 | qsp 100% (v/v) |
| - Insuline | Sigma, H-0888 | 0,5 µg / ml |
| - EGF | Sigma, E-4127 | 10 ng / ml |
| - B-FGF | Gibco, 13256-029 | 10 ng / ml |
| - PdBu | Sigma, P-1269 | 0,25 µg / ml |
| - Chlorure de calcium | Prolabo, 22317297 | 1,8 mM |

Ce milieu est renouvelé toutes les 48 heures pendant une à deux semaines.

Après cette sélection des mélanocytes, on favorise la prolifération des MHN, en remplaçant le Milieu 6 par le Milieu 7.

Le Milieu 7 est composé de 10 % de Milieu C et 90 % de Milieu A décrit précédemment, complémenté par PdBu (Sigma, P-1269) dont la concentration finale dans le milieu est de 0,25 µg / ml.

| **COMPOSITION DU MILIEU C** | | |
|---|---|---|
| Composés | Fournisseur, référence | Concentration finale dans le milieu |
| - Milieu E-199 | Gibco, 31150-022 | qsp 100% (v/v) |
| - Sérum de veau foetal | Gibco, 10099-133 | 10 % (v/v) |
| - L-glutamine | Gibco, 25030-024 | 2 mM |
| - IBMX | Sigma, I-5879 | 10⁻⁴ M |
| - Génécitine | Gibco, 066-01811Y | 100 µg/ml |

A ce stade, la population cellulaire obtenue est pure. Elle est constituée exclusivement de MHN.

Les MHN sont ensemencés en microplaques 96 puits (Falcon, Franklin Lakes, NJ, USA) dans du Milieu 6. Après 24h, le milieu est remplacé par du Milieu 8 qui servira pendant toute la fin de cette étude. La composition du Milieu 8 est décrite ci-dessous.

| **COMPOSITION DU MILIEU 8** | | |
|---|---|---|
| Composés | Fournisseur, référence | Concentration finale dans le milieu |
| - Milieu MCDB-153 | Sigma, M-7403 | qsp 100% (v/v) |
| - Sérum de veau foetal | Gibco, 10099-133 | 2 % (v/v) |
| - Insuline | Sigma, I-6634 | 5 µg / ml |
| - Transferrine | Sigma | 10 µg / ml |
| - α-Tocophérol | Sigma, T1157 | 1 µg / ml |
| - Pénicilline/Streptomycine | Sigma, P4333 | 0,4 % (v/v) |

L'oligonucléotide SEQ ID NO.2 est ajouté extemporanément dans le milieu d'étude, à des concentrations de 50 nM, 100 nM, 250 nM, 500 nM, 1µM. Les traitements sont effectués chaque jour pendant 7 jours. Pour chaque concentration, 6 essais sont réalisés.

### Mesure de vitesses de réaction de l'activité dopa-oxydase :

A la fin de ces traitements répartis sur 7 jours, des mesures de l'activité dopa-oxydase sont réalisées. Ces mesures sont pratiquées de la même manière qu'à l'exemple 2. Les vitesses de réaction sont calculées et exprimées en 10⁻⁴ Unité DO/mn.

Les résultats obtenus pour les cultures traitées par l'oligonucléotide SEQ ID NO.2 et pour les cultures témoin non traitées figurent dans le tableau 3 (σ : écart type).

**TABLEAU 3**

| Concentrations de l'oligonucléotide SEQ ID NO. 2 | Vitesses de réaction | Vitesses de réaction (témoin sans oligonucléotide) | % de variation par rapport au témoin |
|---|---|---|---|
| 50 nM | 33,8 σ = 3,4 | 33,0 σ = 3,5 | +2,4 |
| 100 nM | 29,8 σ = 1,3 | 33,0 σ = 3,5 | -9,7 |
| 250 nM | 29,6 σ = 2,2 | 33,0 σ = 3,5 | -10,3 |
| 500 nM | 28,3 σ = 1,5 | 33,0 σ = 3,5 | -14,2 |
| 1 µM | 27,6 σ = 2,9 | 33,0 σ = 3,5 | -16,3 |

On observe d'après les résultats ci-dessus, qu'à partir d'une concentration assez faible en oligonucléotide (100 nM) et au dessus de cette concentration, la vitesse de réaction de l'activité dopa-oxydase diminue très sensiblement.

Ainsi, comme cela a déjà été expliqué en conclusion de l'exemple 2, les oligonucléotides selon l'invention réduisent la capacité de synthèse de mélanine par les mélanocytes. Il est demontré dans le présent test que cette réduction est indépendante des caractéristiques génétiques de la personne concernée.

Dans le présent exemple, s'agissant d'un donneur mulâtre présentant une pigmentation de base correspondant à une couleur de peau foncée, l'utilisation d'un oligonucléotide selon l'invention, conduira à un effet blanchissant, c'est-à-dire à un éclaircissement de la teinte de la peau.

### Exemple 4 : Etude de l'inhibition de la mélanogenèse UV-induite, sur des mélanocytes humains normaux par un oligonucléotide selon l'invention

A la suite des résultats des exemples précédents, l'objectif du présent test est de vérifier si les oligonucléotides de l'invention sont également actifs dans le cas où les mélanocytes sont activés par une stimulation, en l'occurrence par une irradiation ultra-violette UVB (280-320 nm). Autrement dit, il s'agit d'évaluer la capacité des oligonucléotides à réduire l'activité de la dopa-oxydase de mélanocytes indépendamment de la stimulation de la mélanogénèse.

A cet effet, le test porte sur des mélanocytes d'un donneur adulte de type caucasien. Les mélanocytes utilisés ici sont obtenus à partir de peau de plastie mammaire. La technique d'obtention de ces mélanocytes est la même que celle utilisée et décrite dans l'exemple 3.

L'expérience est réalisée en boîtes 35 mm. Les mélanocytes sont ensemencés en boîtes 35 mm (Falcon, Franklin Lakes, NJ, USA) à raison de 250000 cellules et 2 ml de Milieu 8 par boîte. La composition du Milieu 8 est décrite à l'exemple 3. Les traitements avec l'oligonucléotide SEQ ID NO.2 ainsi que les irradiations UVB (Bio-Energie, Vilbert-Lourmat, Marne-la-Vallée, France) à 8 mJ/cm² commencent 24h après l'ensemencement.

Avant chaque irradiation UVB, les MHN sont placés dans du PBS (2ml/boîte). Après irradiation, les cellules sont replacées dans 2 ml de Milieu 8 contenant ou non l'oligonucléotide SEQ ID NO.2.

L'oligonucléotide SEQ ID NO.2 est ajouté extemporanément dans le milieu d'étude (Milieu 7 de l'exemple 3), à des concentrations de 100 nM, 250 nM et 500 nM.

Ces traitements avec l'oligonucléotide sont effectués chaque jour pendant 9 jours. Les irradiations ont lieu chaque jour, sauf les jours 5 et 6. Pour chaque concentration, 3 essais sont réalisés.

### Mesure de vitesses de réaction de l'activité dopa-oxydase :

A la fin de ces traitements, à savoir 9 jours de traitement avec l'oligonucléotide et 7 irradiations, des mesures de l'activité dopa-oxydase sont réalisées.

A cet effet, les MHN sont décollées des boîtes 35 mm à l'aide de 500 µl de trypsine 0,1% - EDTA 0,05%, puis reprises dans 1,5 ml de milieu E-199 sans rouge de phénol (Gibco) et supplémentés par 10 % de sérum de veau foetal. Une portion aliquote de cette suspension cellulaire est utilisée pour un dénombrement cellulaire une autre est centrifugée et le culot de mélanocytes obtenu est repris dans 50 µl de tampon de lyse pour la mesure de la vitesse de réaction de la dopa-oxydase comme décrit exemple 2.

Les vitesses de réaction de l'activité dopa-oxydase sont calculées et exprimées en 10⁻⁴ Unité DO / mn / 10⁶ MHN. Les résultats obtenus sont présentés dans la tableau 4 (σ: écart-type).

**TABLEAU 4**

| Traitements des cultures de cellules: irradiation UVB / concentration en oligonucléotides SEQ ID NO.2 | Vitesse de réaction | % de variation par rapport au témoin non irradié | % de variation par rapport au témoin irradié |
|---|---|---|---|
| Non irradiation / aucun oligonucléotide (témoin-non irradié) | 368,2 σ = 19,9 | 0 | |
| Irradiation / aucun oligonucléotide (témoin irradié) | 409,1 σ = 32,6 | + 11,1 % | 0 |
| Irradiation / 100 nM | 328,1 σ = 44,8 | | - 19,8 % |
| Irradiation / 250 nM | 325,3 σ = 43,1 | | - 20,5 % |
| Irradiation / 500 nM | 327,4 σ = 49,5 | | - 20 % |

Les résultats ci-dessus démontrent clairement que les oligonucléotides selon l'invention, diminuent très sensiblement la vitesse de réaction de l'activité dopa-oxydase dans le cas de mélanocytes stimulés sous l'effet d'un rayonnement UVB.

Ainsi les oligonucléotides de l'invention diminuent non seulement la capacité des mélanocytes à l'état d'activité basale à synthétiser de la mélanine, comme cela a été montré dans les exemples précédents, mais ils diminuent aussi cette capacité pour les mélanocytes à l'état activé. Ces oligonucléotides pourront donc être également utilisés en particulier pour prévenir ou atténuer une pigmentation déclenchée par le rayonnement ultra-violet présent dans la lumière solaire.

### Exemple 5 : Poudre pour l'éclaircissement du teint du visage

| | |
|---|---|
| Microcellulose | 20,00% |
| Sodium lauryl sulfoacetate | 15,00% |
| Oligonucléotide (SEQ ID NO.5) | 1,00% |
| (phosphodiester) | |
| Parfum, colorants, conservateurs | qs. |
| Talc | Qsp. 100% |

Cette poudre présente une double action. Elle permet un nettoyage de la peau, et de plus elle permet, par un usage régulier durant quelques jours, d'éclaircir le teint. On peut l'appliquer sur la peau du visage une à deux fois par jour.

### Exemple 6 : Emulsion-gel visage de jour dépigmentante

| | |
|---|---|
| Glycérine | 5,00% |
| triglycérides | 5,00% |
| caprylique/caprique/succinique | |
| Méthoxycinnamate d'octyle | 1,00% |
| Diméthicone copolyol | 0,50% |
| Acrylates / C10-30 alkyl acrylate | 0,50% |
| crosspolymer | |
| Oligonucléotide SEQ ID NO.3 | 0,01% |
| (phosphodiester) | |
| Neutralisant | qs. |
| Conservateurs, parfum, colorants | qs. |
| Eau | qsp. 100% |

Certaines personnes soumises au rayonnement plus ou moins intense de la lumière du jour, voire du soleil directement, souhaitent conserver un teint clair et éviter l'apparition de taches pigmentées. L'utilisation de l'émulsion-gel ci-dessus permettra d'atteindre ce but. Cette composition s'applique sur le visage généralement le matin. Elle agit aussi bien de manière préventive que curative sur la pigmentation, régulière ou non, du visage.

### Exemple 7 : Fluide protecteur SPF 30 préventif des taches pigmentaires

| | |
|---|---|
| Pentacyclométhicone volatile | 49,00% |
| Dioxyde de titane | 15,00% |
| Méthoxycinnamate d'octyle | 7,50% |
| Glycérine | 5,00% |
| Phényltriméthicone | 5,00% |
| Diméthicone copolyol | 3,00% |
| Polyméthylméthacrylate | 2,50% |
| Butyl méthoxydibenzoyle méthane | 1,00% |
| Oligonucléotide SEQ ID NO.2 | 0,1% |
| (phosphodiester) | |
| Neutralisant, parfum, conservateurs, | qs. |
| antioxydants | |
| Eau | qsp. 100% |

Cette composition est à utiliser pour prévenir l'apparition de taches pigmentaires, chez les personnes prédisposées à ce phénomène, avant l'exposition à un rayonnement solaire intense. Il est à noter que la présence d'une concentration élevée en filtre solaire permet de compenser la diminution de la protection naturelle, conséquence de la baisse du taux de mélanine.

### Exemple 8 : Crème visage dépigmentante

| | |
|---|---|
| Glycéryl stéarate + Peg-100 stéarate | 5,00% |
| Polyisobutène hydrogéné | 4,00% |
| Magnésium ascorbyl phosphate | 3,30% |
| Tricaprylate /caprate de glycérol | 3,00% |
| Squalane | 3,00% |
| Glycérine | 2,00% |
| Cire d'abeille | 1,50% |
| Cétéaryl octanoate | 1,50% |
| Alcool cétylique | 1,00% |
| Alcool stéarylique | 1,00% |
| Diméthicone | 1,00% |
| Gomme xanthane | 0,30% |
| Acide éthylène diamine tétracétique | 0,20% |
| Acide citrique | 0,10% |
| Citrate de sodium | 0,10% |
| Oligonucléotide SEQ ID NO.6 | 0,10% |
| (phosphodiester) | |
| Neutralisant, Parfum, Conservateurs | qs. |
| Eau | qsp. 100% |

L'utilisation de cette crème permet de traiter les irrégularités de la pigmentation cutanée, en atténuant ou supprimant les taches de sénescence ou les taches pigmentaires actiniques. Elle homogénéise la coloration de la peau et éclaircit le teint.

### Exemple 9 : Lotion visage pour éclaircir le teint

| | |
|---|---|
| Alcool éthylique | 30,00% |
| PPG-3 Myristyl éther | 5,00% |
| Glycérine | 2,00% |
| Carbomer | 0,20% |
| Polysorbate 20 | 0,20% |
| Oligonucléotide SEQ ID NO.4 | 0,01% |
| (phosphorothioate) | |
| Neutralisant, Parfum, Conservateurs | qs. |
| Eau | qsp. 100% |

Cette lotion pour éclaircir le teint s'utilise après le démaquillage et le nettoyage de la peau.

### Exemple 10 : Sérum Visage éclaircissant

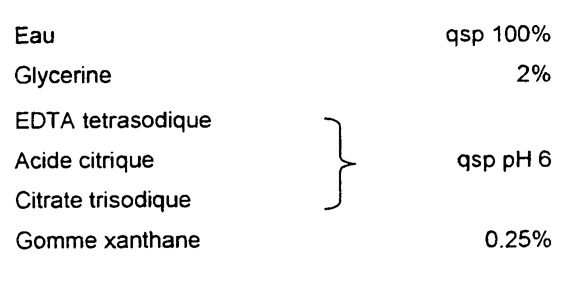

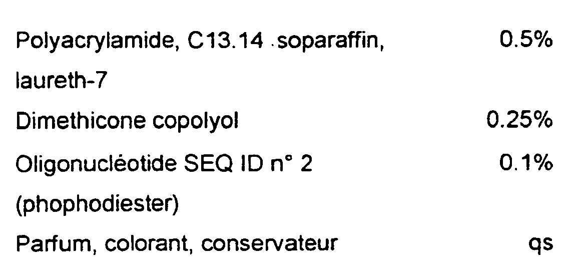

Une goutte de cette composition très concentrée de sérum, s'applique sur le visage généralement avant l'application d'une crème pour le visage. Ce sérum s'utilise habituellement par cures d'une à deux semaines pour obtenir ou entretenir un éclaircissement du teint.

### Exemple 11 : Lotion capillaire pour éclaircir la chevelure

| | |
|---|---|
| Eau | qsp 100% |
| Alcool | 50% |
| Panthenylethyl ether | 0.5% |
| Acétate DL-α-tocopherol | 0.2% |
| Polysorbate 60 | 1 % |
| Oligonucléotide SEQ ID NO.1 | 0.01% |
| (phosphorothioate) | |
| Parfum | 0.2% |
| Glycerine | 0.5% |
| Colorant | qs |

Cette lotion s'applique sur les cheveux matin et soir pendant la durée nécessaire pour obtenir un éclaircissement progressif de la chevelure. Cette durée est généralement de plusieurs semaines.

### Exemple 12 : Crème main (gel-crème anti-taches) pour les mains

| | |
|---|---|
| Caprilic/capric diglyceryl succinate | 6% |
| Octyl octanoate | 2.5% |
| Methoxycinnamate d'octyle | 6% |
| Oligonucléotide SEQ ID NO. 5 | 0.001% |
| (phosphodiester) | |
| Phenyltriméticone | 2.5% |
| Benzophenon-3 | 0.5% |
| Hyaluronate de sodium | 0.05% |
| Gomme xanthane | 0.2% |
| Acrylates/C10.30 alkyl acrylate | 0.5% |
| copolymer | |
| Glycerine | 2% |
| PEG 150 | 3% |
| Neutralisants, Colorants, parfum, | qs |
| conservateurs | |
| Eau purifiée | qsp 100% |

Cette crème doit être appliquée directement sur les taches de sénescence (lentigos séniles) des mains, pour en atténuer la coloration.

## Revendications

1. Oligonucléotide comportant un nombre de nucléotides compris entre 18 et 25, de préférence égal à 20, capable de s'hybrider de façon spécifique avec le gène ou un produit du gène codant pour la tyrosinase, **caractérisé en ce que** l'oligonucléotide inhibe l'expression de la tyrosinase et présente au moins 80% d'identité avec l'une des séquences SEQ ID N° 6 à N° 10 ayant la signification suivante :
- SEQ ID N° 6 5'-AGGAACTGGCTAATTGGAGT-3'
- SEQ ID N° 7 5'-CAAGGTCTGCAGGAACTGGC-3'
- SEQ ID N° 8 5'-CCTCACAAGGTCTGCAGGAA-3'
- SEQ ID N° 9 5'-CTACAGACAATCTGCCAAGA-3'
- SEQ ID N° 10 5'-GCATTCTTCCTCTAGTCCTC-3'

2. Oligonucléotide en tant que produit nouveau dont la séquence est l'une des séquences ID N° 6 à N° 10 ayant la signification suivante :
- SEQ IDN° 6 5'-AGGAACTGGCTAATTGGAGT-3'
- SEQ ID N° 7 5'-CAAGGTCTGCAGGAACTGGC-3'
- SEQ ID N° 8 5'-CCTCACAAGGTCTGCAGGAA-3'
- SEQ ID N° 9 5'-CTACAGACAATCTGCCAAGA-3'
- SEQ ID N° 10 5'-GCATTCTTCCTCTAGTCCTC-3'

3. Oligonucléotide selon l'une des revendications précédentes, comprenant une ou plusieurs modifications chimiques au niveau de ses parties sucres, ses parties nucléobases ou son squelette internucléotidique, lesdites modifications conférant des caractéristiques physico-chimiques souhaitables telles qu'une biodisponibilité accrue, l'augmentation de l'affinité pour les séquences cibles, l'augmentation de l'internalisation cellulaire ou une meilleure stabilité biologique ou l'augmentation de la stabilité en présence de nucléases cellulaires.

4. Oligonucléotide selon la revendication 3, **caractérisé en ce que** sa partie sucre comprend un substituant 2'-O-fluoro ou 2'-O-alkyle, préférentiellement un substituant 2'-O-éthyloxyméthyle ou 2'-O-méthyle.

5. Oligonucléotide selon la revendication 3 ou 4, **caractérisé en ce qu'**une partie des groupements phosphodiesters de son squelette internucléotidique est remplacée par des groupements phophorothioates.

6. Oligonucléotide selon la revendication 3 ou 4, **caractérisé en ce qu'**une partie des groupements phosphodiesters de son squelette internucléotididique est remplacée par des groupements méthylphosphonates.

7. Oligonucléotide selon la revendication 3 ou 4, **caractérisé en ce que** tous les groupements phosphodiesters sont remplacés par des groupements phophorothioates.

8. Oligonucléotide selon la revendication 3 ou 4, **caractérisé en ce que** tous les groupements phosphodiesters sont remplacés par des groupements méthylphosphonates.

9. Oligonucléotide selon la revendication 3 ou 4, **caractérisé en ce que** les groupements phosphodiesters sont remplacés en tout ou partie par des groupements phosphorothioates et/ou par des groupements méthylphosphonates.

10. Oligonucléotide selon l'une des revendications 1 à 9 auquel on a greffé un vecteur d'administration linéaire de type acide nucléique ou peptidique, ou un vecteur d'administration circulaire de type plasmidique.

11. Oligonucléotide selon l'une des revendications précédentes en tant que médicament.

12. Composition cosmétique ou dermatologique contenant au moins un oligonucléotide selon l'une des revendications 1 à 11 et un milieu cosmétiquement ou dermatologiquement acceptable.

13. Composition cosmétique ou dermatologique selon la revendication 12 contenant un ou plusieurs agents actifs choisis parmi l'acide ellagique et ses dérivés ; le résorcinol et ses dérivés ; la vitamine C et ses dérivés ; le pantothénate sulfonate et ses dérivés ; des molécules interférant directement ou indirectement avec l'alpha-mélanocyte stimulating hormone (α-MSH) ou son récepteur ou l'hormone adrénocorticotropique (ACTH) ; les polyols tels que la glycérine le glycol ou le propylène glycol ; les vitamines ; les agents kératolytiques et/ou desquamants tels que l'acide salicylique et ses dérivés ; les alpha-hydroxyacides tels que l'acide lactique ou l'acide malique, seuls ou greffés ; l'acide ascorbique et ses dérivés ; l'acide rétinoïque ; le rétinaldéhyde ; le rétinol et ses dérivés tels que le palmitate, le propionate ou l'acétate, en préparation liposomique ou non ; des agents antiglycations et/ou antioxydants pris seuls ou en association tels que le tocophérol et ses dérivés, la thiotaurine, l'hypotaurine, l'aminoguanidine, la thiamine pyrophosphate, la pyridoxamine, la lysine, l'histidine, l'arginine, la phénylalanine, la pyridoxine, l'adénosine triphosphate; les agents anti-inflammatoires tels que les stéaryl glycyrrhétinate ; les agents apaisants et leurs mélanges, les filtres solaires chimiques ou physiques tels que le méthoxycinnamate d'octyle, le butyl-méthoxydibenzoyl-méthane, l'oxyde de titane et l'oxyde de zinc micronisés ; et les acides désoxyribonucléiques ou nucléiques.

14. Composition selon la revendications 12 ou 13, **caractérisé en ce que** le/les oligonucléotides représentent 0,00001 % à 10 %, de préférence 0,0003 % à 3 % du poids total de la composition.

15. Composition cosmétique ou dermatologique selon l'une des revendications 13 et 14, **caractérisée en ce qu'**elle se présente sous forme habituellement utilisée pour une application topique et notamment sous forme d'une solution aqueuse, hydroalcoolique ou huileuse, d'une émulsion huile dans eau ou eau dans l'huile ou multiple, d'un gel aqueux ou huileux, d'un produit anhydre liquide, pâteux ou solide, d'une dispersion de particules polymériques, telles que des nanosphères et des nanocapsules, dans une phase aqueuse ou huileuse, ou encore d'une dispersion de vésicules lipidiques de type ionique ou non-ionique.

16. Utilisation comme agent cosmétique d'un olignonucléotide selon l'une quelconque des revendications 1 à 10.

17. Utilisation d'un oligonucléotide selon la revendication 16 pour dépigmenter ou blanchir la peau, les poils ou les cheveux humains.

18. Utilisation d'un oligonucléotide selon l'une des revendications 1 à 11 pour la fabrication d'un médicament destiné au traitement ou à la prévention des maladies se traduisant par la surexpression de la tyrosinase, par voie topique.

19. Utilisation d'un oligonucléotide selon l'une quelconque des revendications 1 à 11 pour la fabrication d'un médicament pour le traitement ou la prévention des hyperpigmentations régionales par hyper-activité mélanocytaire telles que les mélasmas idiophatiques, des hyperpigmentations localisées par hyper-activité et prolifération mélanocytaire bénigne telles que les taches pigmentaires de sénescence (lentigo seniles), des hyperpigmentations accidentelles telles que la photosensibilisation ou l'hyperpigmentation cicatricielle, et pour le traitement de certaines leucodermies telles que le vitiligo.

20. Utilisation d'un oligonucléotide selon l'une quelconque des revendications 18 et 19 pour la fabrication d'un médicament destiné à être administré de façon simultanée, séparée ou étalée dans le temps en association avec un ou plusieurs agents actifs choisis parmi l'acide ellagique et ses dérivés ; l'hydroquinone; l'arbutine ; le résorcinol et ses dérivés; la vitamine C et ses dérivés; le pantothénate sulfonate et ses dérivés; l'acide kojique; les extraits placentaires ; des molécules interférant directement ou indirectement avec l'alpha-mélanocyte stimulating hormone (α-MSH) ou son récepteur ou l'hormone adrénocorticotropique (ACTH) ; les polyols tels que la glycérine le glycol ou le propylène glycol ; les vitamines ; les agents kératolytiques ou desquamants tels que l'acide salicylique et ses dérivés; les alpha-hydroxyacides tels que l'acide lactique ou l'acide malique, seuls ou greffés ; l'acide ascorbique et ses dérivés ; l'acide rétinoïque, le rétinaldéhyde, le rétinol et ses dérivés tels que le palmitate, le propionate ou l'acétate, en préparation liposomique ou non; des agents antiglycations ou antioxydants pris seuls ou en association tels que le tocophérol et ses dérivés, la thiotaurine, l'hypotaurine, l'aminoguanidine, la thiamine pyrophosphate, la pyridoxamine, la lysine, l'histidine, l'arginine, la phénylalanine, la pyridoxine, l'adénosine triphosphate ; les agents anti-inflammatoires tels que le stéaryl glycyrrhétinate ; les agents apaisants et leurs mélanges, les filtres solaires chimiques ou physiques tels que le méthoxycinnamate d'octyle, le butyl-méthoxydibenzoyl-méthane, l'oxyde de titane et l'oxyde de zinc micronisé ; et les acides désoxyribonucléiques ou nucléiques.

## Claims

1. Oligonucleotide comprising a number of nucleotides of between 18 and 25, preferably equal to 20, capable of hybridizing specifically with the gene or a product of the gene encoding tyrosinase, **characterized in that** the oligonucleotide inhibits the expression of the tyrosinase and exhibits at least 80% identity with one of the sequences SEQ ID No. 6 to No. 10 which signify the following:
- SEQ ID NO.6 5'-AGGAACTGGCTAATTGGAGT-3'
- SEQ ID NO.7 5'-CAAGGTCTGCAGGAACTGGC-3'
- SEQ ID NO.8 5'-CCTCACAAGGTCTGCAGGAA-3'
- SEQ ID NO.9 5'-CTACAGACAATCTGCCAAGA-3'
- SEQ ID NO.10 5'-GCATTCTTCCTCTAGTCCTC-3'

2. Oligonucleotide as a novel product, the sequence of which is one of the sequences SEQ ID No. 6 to SEQ ID No. 10 which signify the following:
- SEQ ID NO.6 5'-AGGAACTGGCTAATTGGAGT-3'
- SEQ ID NO.7 5'-CAAGGTCTGCAGGAACTGGC-3'
- SEQ ID NO.8 5'-CCTCACAAGGTCTGCAGGAA-3'
- SEQ ID NO.9 5'-CTACAGACAATCTGCCAAGA-3'
- SEQ ID NO.10 5'-GCATTCTTCCTCTAGTCCTC-3'

3. Oligonucleotide according to one of the preceding claims, comprising one or more chemical modifications to its sugar components, its nucleobase components or its internucleotide backbone, said modifications conferring desirable physicochemical characteristics, such as increased bioavailability, an increase in the affinity for the target sequences, an increase in cellular internalization, or better biological stability or an increase in stability in the presence of cellular nucleases.

4. Oligonucleotide according to Claim 3,
**characterized in that** its sugar component comprises a 2'-O-fluoro or 2'-O-alkyl substituent, preferably a 2'-O-ethyloxymethyl or 2'-O-methyl substituent.

5. Oligonucleotide according to Claim 3 or 4, **characterized in that** some of the phosphodiester groups of its internucleotide backbone are replaced with phosphorothioate groups.

6. Oligonucleotide according to Claim 3 or 4, **characterized in that** some of the phosphodiester groups of its internucleotide backbone are replaced with methyl phosphonate groups.

7. Oligonucleotide according to Claim 3 or 4, **characterized in that** all the phosphodiester groups are replaced with phosphorothioate groups.

8. Oligonucleotide according to Claim 3 or 4, **characterized in that** all the phosphodiester groups are replaced with methyl phosphonate groups.

9. Oligonucleotide according to Claim 3 or 4, **characterized in that** the phosphodiester groups are entirely or partly replaced with phosphorothioate groups and/or methyl phosphonate groups.

10. Oligonucleotide according to one of Claims 1 to 9, onto which a linear administration vector of the nucleic acid or peptide type or a circular administration vector of the plasmid type has been grafted.

11. Oligonucleotide according to one of the preceding claims, as a medicinal product.

12. Cosmetic or dermatological composition containing at least one oligonucleotide according to one of Claims 1 to 11 and a cosmetically or dermatologically acceptable medium.

13. Cosmetic or dermatological composition according to Claim 12, containing one or more active agents chosen from ellagic acid and derivatives thereof; resorcinol and derivatives thereof; vitamin C and derivatives thereof; pantothenate sulphonate and derivatives thereof; molecules which interfere directly or indirectly with alpha-melanocyte-stimulating hormone (α-MSH) or its receptor or adrenocorticotropic hormone (ACTH); polyols, such as glycerol, glycol or propylene glycol; vitamins; keratolytic or desquamating agents, such as salicylic acid and derivatives thereof; α-hydroxy acids, such as lactic acid or malic acid, alone or grafted; ascorbic acid and derivatives thereof; retinoic acid; retinaldehyde; retinol and derivatives thereof, such as palmitate, propionate or acetate, which may or may not be in a liposomal preparation; antiglycation agents or antioxidants, taken alone or in combination, such as tocopherol and derivatives thereof, thiotaurine, hypotaurine, aminoguanidine, thiamine pyrophosphate, pyridoxamine, lysine, histidine, arginine, phenylalanine, pyridoxine, adenosine triphosphate; anti-inflammatory agents, such as stearyl glycyrrhetinate; soothing agents and mixtures thereof, chemical or physical sunscreens, such as micronized zinc oxide, titanium oxide, butylmethoxydibenzoylmethane and octyl methoxycinnamate; and deoxyribonucleic or nucleic acids.

14. Composition according to Claim 12 or 13, **characterized in that** the oligonucleotide(s) represent(s) 0.00001% to 10%, preferably 0.0003% to 3% of the total weight of the composition.

15. Cosmetic or dermatological composition according to either of Claims 13 and 14, **characterized in that** it is provided in a form conventionally used for topical application, and in particular in the form of an aqueous, aqueous-alcoholic or oily solution, of an oil-in-water, water-in-oil or multiple emulsion, of an aqueous or oily gel, of a liquid, pasty or solid anhydrous product, of a dispersion of polymeric particles, such as nanospheres and nanocapsules, in an aqueous or oily phase, or of a dispersion of lipid vesicles of the ionic or nonionic type.

16. Use, as a cosmetic agent, of an oligonucleotide according to any one of Claims 1 to 10.

17. Use of an oligonucleotide according to Claim 16, for depigmenting or bleaching human skin, body hair or head hair.

18. Use of an oligonucleotide according to one of Claims 1 to 11, for the manufacture of a medicinal product intended for the treatment or for the prevention of diseases resulting in the overexpression of tyrosinase, via the topical route.

19. Use of an oligonucleotide according to any one of Claims 1 to 11, for the manufacture of a medicinal product for the treatment or the prevention of regional hyperpigmentations due to melanocytic hyperactivity, such as idiopathic melasmas, of localized hyperpigmentations due to benign melanocytic hyperactivity and proliferation, such as senescent pigmentary blemishes (senile lentigo), and of accidental hyperpigmentations, such as photo-sensitization or cicatricial hyperpigmentation, and for the treatment of certain leukodermias, such as vitiligo.

20. Use of an oligonucleotide according to either of Claims 18 and 19, for the manufacture of a medicinal product intended to be administered simultaneously, separately or in a way which is spread out over time, in combination with one or more active agents chosen from ellagic acid and derivatives thereof; hydroquinone; arbutin; resorcinol and derivatives thereof; vitamin C and derivatives thereof; pantothenate sulphonate and derivatives thereof; kojic acid; placental extracts; molecules which interfere directly or indirectly with alpha-melanocyte-stimulating hormone (α-MSH) or its receptor or adrenocorticotropic hormone (ACTH); polyols, such as glycerol, glycol or propylene glycol; vitamins; keratolytic or desquamating agents, such as salicylic acid and derivatives thereof; α-hydroxy acids, such as lactic acid or malic acid, alone or grafted; ascorbic acid and derivatives thereof; retinoic acid; retinaldehyde; retinol and derivatives thereof, such as palmitate, propionate or acetate, which may or may not be in a liposomal preparation; antiglycation agents or antioxidants, taken alone or in combination, such as tocopherol and derivatives thereof, thiotaurine, hypotaurine, aminoguanidine, thiamine pyrophosphate, pyridoxamine, lysine, histidine, arginine, phenylalanine, pyridoxine, adenosine triphosphate; anti-inflammatory agents, such as stearyl glycyrrhetinate; soothing agents and mixtures thereof, chemical or physical sunscreens, such as micronized zinc oxide, titanium oxide, butylmethoxydibenzoylmethane and octyl methoxycinnamate; and deoxyribonucleic or nucleic acids.

## Patentansprüche

1. Oligonukleotid, umfassend eine Zahl von Nukleotiden, die zwischen 18 und 25, bevorzugt 20, einschließlich liegt, welches in der Lage ist, spezifisch mit dem Gen oder einem Produkt des Gens, das für Tyrosinase kodiert, zu hybridisieren, **dadurch gekennzeichnet, dass** das Oligonukleotid die Tyrosinase-Expression inhibiert und mindestens 80% Identität mit einer der Sequenzen SEQ ID N° 6 bis N° 10 aufweist, welche die folgende Bedeutung haben:
- SEQ ID N° 6 5'-AGGAACTGGCTAATTGGAGT-3'
- SEQ ID N° 7 5'-CAAGGTCTGCAGGAACTGGC-3'
- SEQ ID N° 8 5'-CCTCACAAGGTCTGCAGGAA-3'
- SEQ ID N° 9 5'-CTACAGACAATCTGCCAAGA-3'
- SEQ ID N° 10 5'-GCATTCTTCCTCTAGTCCTC-3'

2. Oligonukleotid als neues Produkt, dessen Sequenz eine der Sequenzen ID N° 6 bis N° 10 ist, welche die folgende Bedeutung haben:
- SEQ IDN° 6 5'-AGGAACTGGCTAATTGGAGT-3'
- SEQ ID N° 7 5'-CAAGGTCTGCAGGAACTGGC-3'
- SEQ ID N° 8 5'-CCTCACAAGGTCTGCAGGAA-3'
- SEQ ID N° 9 5'-CTACAGACAATCTGCCAAGA-3'
- SEQ ID N° 10 5'-GCATTCTTCCTCTAGTCCTC-3'

3. Oligonukleotid nach einem der vorangehenden Ansprüche, umfassend eine oder mehrere chemische Modifikationen in seinen Zuckerteilen, seinen Nukleobasenteilen oder seinem Internukleotid-Gerüst, wobei diese Modifikationen wünschenswerte physikochemische Eigenschaften verleihen, wie eine erhöhte Bioverfügbarkeit, die Erhöhung der Affinität zu den Zielsequenzen, die Erhöhung der zellulären Aufnahme oder eine bessere biologische Stabilität oder die Erhöhung der Stabilität in Anwesenheit von zellulären Nukleasen.

4. Oligonukleotid nach Anspruch 3, **dadurch gekennzeichnet, dass** sein Zuckerteil einen 2'-O-Fluor- oder 2'-O-Alkylsubstituenten, bevorzugt einen 2'-O-Ethyloxymethyl- oder 2'-O-Methylsubstituenten umfasst.

5. Oligonukleotid nach Anspruch 3 oder 4, **dadurch gekennzeichnet, dass** ein Teil der Phosphodiestergruppen seines Internukleotid-Gerüsts durch Phosphorothioatgruppen ersetzt ist.

6. Oligonukleotid nach Anspruch 3 oder 4, **dadurch gekennzeichnet, dass** ein Teil der Phosphodiestergruppen seines Internukleotid-Gerüsts durch Methylphosphonatgruppen ersetzt ist.

7. Oligonukleotid nach Anspruch 3 oder 4, **dadurch gekennzeichnet, dass** alle Phosphodiestergruppen durch Phosphorothioatgruppen ersetzt sind.

8. Oligonukleotid nach Anspruch 3 oder 4, **dadurch gekennzeichnet, dass** alle Phosphodiestergruppen durch Methylphosphonatgruppen ersetzt sind.

9. Oligonukleotid nach Anspruch 3 oder 4, **dadurch gekennzeichnet, dass** die Phosphodiestergruppen ganz oder teilweise durch Phosphorothioatgruppen und/oder Methylphosphonatgruppen ersetzt sind.

10. Oligonukleotid nach einem der Ansprüche 1 bis 9, auf welches ein linearer Verabreichungsvektor vom Typ Nuklein- oder Peptidsäure oder ein ringförmiger Verabreichungsvektor der plasmidischen Art gepfropft wurde.

11. Oligonukleotid nach einem der vorangehenden Ansprüche als Medikament.

12. Kosmetische oder dermatologische Zusammensetzung, welche mindestens ein Oligonukleotid nach einem der Ansprüche 1 bis 11 und ein kosmetisch oder dermatologisch annehmbares Medium enthält.

13. Kosmetische oder dermatologische Zusammensetzung nach Anspruch 12, welche einen oder mehrere Wirkstoffe enthält, die ausgewählt sind aus Ellagsäure und deren Derivaten; Resorcin und dessen Derivaten; Vitamin C und dessen Derivaten; Pantothenatsulfonat und dessen Derivaten; Molekülen, die direkt oder indirekt mit alpha-Melanozyten-stimulierendem Hormon (α-MSH) oder dessen Rezeptor oder adrenokortikotropem Hormon (ACTH) wechselwirken; Polyolen, wie Glycerin, Glycol oder Propylenglycol; Vitaminen; keratolytischen und/oder schuppenlösenden Wirkstoffen, wie Salicylsäure und deren Derivaten; alpha-Hydroxysäuren, wie Milchsäure oder Äpfelsäure, einzeln oder gepfropft; Ascorbinsäure und deren Derivaten; Retinoesäure; Retinaldehyd; Retinol und dessen Derivaten, wie dem Palmitat, Propionat oder Acetat, in Form eines liposomalen oder nicht-liposomalen Präparates; Antiglykierungsmitteln oder Antioxidantien, einzeln oder kombiniert, wie Tocopherol und dessen Derivaten, Thiotaurin, Hypotaurin, Aminoguanidin, Thiaminpyrophosphat, Pyridoxamin, Lysin, Histidin, Arginin, Phenylalanin, Pyridoxin, Adenosintriphosphat; entzündungshemmenden Mitteln, wie Stearylglycyrrhetinat; beruhigenden Mitteln und deren Mischungen, chemischen oder physikalischen Sonnenfiltern, wie Octylmethoxycinnamat, Butylmethoxydibenzoylmethan, mikronisiertem Titandioxid und Zinkoxid; und Desoxyribonukleinsäuren oder Nukleinsäuren.

14. Zusammensetzung nach Anspruch 12 oder 13, **dadurch gekennzeichnet, dass** das bzw. die Oligonukleotide 0,00001 % bis 10 %, bevorzugt 0,0003 % bis 3 % des Gesamtgewichts der Zusammensetzung darstellen.

15. Kosmetische oder dermatologische Zusammensetzung nach einem der Ansprüche 13 und 14, **dadurch gekennzeichnet, dass** sie in einer Form vorliegt, die üblicherweise für eine topische Anwendung verwendet wird, und insbesondere in Form einer wässrigen, wässrig-alkoholischen oder öligen Lösung, einer Öl-in-Wasser- oder Wasser-in-Öl- oder Mehrfach-Emulsion, eines wässrigen oder öligen Gels, eines flüssigen, pastenartigen oder festen wasserfreien Produkts, einer Dispersion von Polymerpartikeln, wie Nanokügelchen und Nanokapseln, in einer wässrigen oder öligen Phase oder auch einer Dispersion von Lipidvesikeln der ionischen oder nichtionischen Art.

16. Verwendung eines Oligonukleotids nach einem der Ansprüche 1 bis 10 als kosmetisches Mittel.

17. Verwendung eines Oligonukleotids nach Anspruch 16 zur Depigmentierung oder Bleichung der menschlichen Haut, Körperhaare oder Haare.

18. Verwendung eines Oligonukleotids nach einem der Ansprüche 1 bis 11 zur Herstellung eines Medikaments, das zur topischen Behandlung oder Vorbeugung von Krankheiten bestimmt ist, die sich in der Überexpression von Tyrosinase äußern.

19. Verwendung eines Oligonukleotids nach einem der Ansprüche 1 bis 11 zur Herstellung eines Medikaments zur Behandlung oder Vorbeugung von lokalen Hyperpigmentierungen, die durch Hyperaktivität der Melanozyten bedingt sind, wie idiopathischen Melasmen, lokalen Hyperpigmentierungen, die durch Hyperaktivität und gutartige Proliferation der Melanozyten bedingt sind, wie Altersflecken (lentigo seniles), akzidentielle Hyperpigmentierungen, wie der Photosensibilisierung oder der Narbenhyperpigmentierung, und zur Behandlung bestimmter Leukoderma, wie Vitiligo.

20. Verwendung eines Oligonukleotids nach einem der Ansprüche 18 und 19 zur Herstellung eines Medikaments, das dazu bestimmt ist, gleichzeitig, getrennt oder gestaffelt in Verbindung mit einem oder mehreren Wirkstoffen verabreicht zu werden, die ausgewählt sind aus Ellagsäure und deren Derivaten; Hydrochinon; Arbutin; Resorcin und dessen Derivaten; Vitamin C und dessen Derivaten; Pantothenatsulfonat und dessen Derivaten; Kojisäure; Plazentaextrakten; Molekülen, die direkt oder indirekt mit alpha-Melanozyten-stimulierendem Hormon (α-MSH) oder dessen Rezeptor oder adrenokortikotropem Hormon (ACTH) wechselwirken; Polyolen, wie Glycerin, Glycol oder Propylenglycol; Vitaminen; keratolytischen oder schuppenlösenden Wirkstoffen, wie Salicylsäure und deren Derivaten; alpha-Hydroxysäuren, wie Milchsäure oder Äpfelsäure, einzeln oder gepfropft; Ascorbinsäure und deren Derivaten; Retinoesäure, Retinaldehyd, Retinol und dessen Derivaten, wie dem Palmitat, Propionat oder Acetat, in Form eines liposomalen oder nicht-liposomalen Präparates; Antiglykierungsmitteln und/oder Antioxidantien, einzeln oder kombiniert, wie Tocopherol und dessen Derivaten, Thiotaurin, Hypotaurin, Aminoguanidin, Thiaminpyrophosphat, Pyridoxamin, Lysin, Histidin, Arginin, Phenylalanin, Pyridoxin, Adenosintriphosphat; entzündungshemmenden Mitteln, wie Stearylglycyrrhetinat; beruhigenden Mitteln und deren Mischungen, chemischen oder physikalischen Sonnenfiltern, wie Octylmethoxycinnamat, Butylmethoxydibenzoylmethan, mikronisiertem Titandioxid und Zinkoxid; und Desoxyribonukleinsäuren oder Nukleinsäuren.


### LISTE DE SÉQUENCES

<110> LVMH RECHERCHE
<120> UTILISATION D'OLIGONUCLÉOTIDES MODULANT L'EXPRESSION DE LA TYROSINASE ET DE LA TYROSINASE-RELATED-PROTEIN-1 COMME AGENTS DÉPIGMENTANTS
<130> D18673
<150> FR 00 01730
   <151> 2000-02-11
<160> 13
<170> PatentIn Ver. 2.2
<210> 1
   <211> 20
   <212> ADN
   <213> Séquence artificielle
<220>
   <223> Oligonucléotide antisens, complémentaire de la séquence nt 111-92 de l'ADN_{c} du gène codant pour la protéine apparentée à la tyrosinase humaine (TRP - Genbank locus HSTYRRP).
<400> 1
   gcaaaacaaa gacctggttt 20
<210> 2
   <211> 20
   <212> ADN
   <213> Séquence artificielle
<220>
   <223> oligonucléotide antisens, complémentaire de la séquence nt 102-83 de l'ADN_{c} du gène codant pour la protéine apparentée à la tyrosinase humaine (TRP - Genbank locus HSTYRRP).
<400> 2
   agacctggtt tgcagctctt 20
<210> 3
   <211> 20
   <212> ADN
   <213> Séquence artificielle
<220>
   <223> Oligonucléotide antisens, complémentaire de la séquence nt 127-108 de l'ADN_{c} du gène codant pour la protéine apparentée à la tyrosinase humaine (TRP - Genbank locus HSTYRRP).
<400> 3
   tgcttgaaat aagagtgcaa 20
<210> 4
   <211> 20
   <212> ADN
   <213> Séquence artificielle
<220>
   <223> Oligonucléotide antisens, complémentaire de la séquence nt 53-34 de l'ADN_{c} du gène codant pour la protéine apparentée à la tyrosinase humaine (TRP - Genbank locus HSTYRRP).
<400> 4
   aaaatccagc tcacaatcct 20
<210> 5
   <211> 20
   <212> ADN
   <213> Séquence artificielle
<220>
   <223> Oligonucléotide antisens, complémentaire de la séquence nt 141-122 de l'ADN_{c} du gène codant pour la protéine apparentée à la tyrosinase humaine (TRP - Genbank locus HSTYRRP).
<400> 5
   aggagcactc attctgcttg 20
<210> 6
   <211> 20
   <212> ADN
   <213> Séquence artificielle
<220>
   <223> Oligonucléotide antisens, complémentaire de la séquence nt 475-457 de l'ADN_{c} du gène codant pour la tyrosinase humaine (Genbank locus HUMTYRA).
<400> 6
   aggaactggc taattggagt 20
<210> 7
   <211> 20
   <212> ADN
   <213> Séquence artificielle
<220>
   <223> Oligonucléotide antisens, complémentaire de la séquence nt 485-466 de l'ADN_{c} du gène codant pour la tyrosinase humaine (Genbank locus HUMTYRA).
<400> 7
   caaggtctgc aggaactggc 20
<210> 8
   <211> 20
   <212> ADN
   <213> Séquence artificielle
<220>
   <223> Oligonucléotide antisens, complémentaire de la séquence nt 490-471 de l'ADN_{c} du gène codant pour la tyrosinase humaine (Genbank locus HUMTYRA).
<400> 8
   cctcacaagg tctgcaggaa 20
<210> 9
   <211> 20
   <212> ADN
   <213> Séquence artificielle
<220>
   <223> Oligonucléotide antisens, complémentaire de la séquence nt 1332-1313 de l'ADN_{c} du gène codant pour la tyrosinase humaine (Genbank locus HUMTYRA).
<400> 9
   ctacagacaa tctgccaaga 20
<210> 10
   <211> 20
   <212> ADN
   <213> Séquence artificielle
<220>
   <223> Oligonucléotide antisens, complémentaire de la séquence nt 506-487 de l'ADN_{c} du gène codant pour la tyrosinase humaine (Genbank locus HUMTYRA).
<400> 10
   gcattcttcc tctagtcctc 20
<210> 11
   <211> 20
   <212> ADN
   <213> Séquence artificielle
<220>
   <223> Oligonucléotide antisens, complémentaire de la séquence nt 5875-5856 de l'ADN codant pour la protéine 1 apparentée à la tyrosinase humaine (TRP1 - Genbank locus AF 001295).
<400> 11
   ttccagtacc tcacaatcct 20
<210> 12
   <211> 20
   <212> ADN
   <213> Séquence artificielle
<220>
   <223> Contrôle antisens de la séquence ID NO.2.
<400> 12
   ttctcgacgt ttggtccaga 20
<210> 13
   <211> 20
   <212> ADN
   <213> Séquence artificielle
<220>
   <223> Combinaison aléatoire de la séquence ID NO.2
<400> 13
   acgtttctcg cctagtgatg 20